# EUROPEAN PATENT SPECIFICATION

(11) **EP 1 876 245 B1**
(45) Date of publication and mention of the grant of the patent: **13.06.2012**
(21) Application number: 07012542.2
(22) Date of filing: 26.06.2007
(51) Int. Cl.: C12Q 1/68

(54) **Method and apparatus for determining the size of a metastatic focus**
Verfahren und Vorrichtung zur Bestimmung der Tumorgröße in Krebsmetastasen
Procédé et appareil pour déterminer la taille des métastases

(30) Priority: 03.07.2006 JP 2006183914
(43) Date of publication of application: 09.01.2008
(73) Proprietor: SYSMEX CORPORATION, Kobe-shi, Hyogo 651-0073 (JP)
(72) Inventor: Motonari, Daito, Kobe-shi, Hyogo 651-0073 (JP); Kazuki, Nakabayashi, Kobe-shi, Hyogo 651-0073 (JP); Yasuhiro, Otomo, Kobe-shi, Hyogo 651-0073 (JP)
(74) Representative: HOFFMANN EITLE

(56) References cited:
- EP-A- 1 669 449
- EP-A- 1 813 682
- EP-A1- 1 508 809
- WO-A-01/51664
- WO-A-2004/045521
- WO-A-2006/066965
- US-A- 6 037 129
- US-A1- 2006 068 418
- US-A1- 2006 121 477

## Description

### FIELD OF THE INVENTION

The present invention relates to a method and an apparatus for determining cancer metastasis contained in a lymph node.

### BACKGROUND

In order to determine to what an extent a cancer has been metastasized to a lymph node tissue, a tissue diagnosis is generally performed. In a tissue diagnosis, a lymph node tissue is sliced thin to prepare a section, a cancer cell in the section is stained using a labeled antibody specifically binding to a cancer cell, and an extent of cancer metastasis is measured by microscopy of this. However, since a lot of skill is needed for a test, the test largely relies on expertise of a tester, and it is difficult to obtain an objective determination result.

As a method for determining metastasis of a cancer cell to a lymph node, for example, the method described in JP2006053113 is known. JP2006053113 describes a method for diagnosing metastasis of pulmonary adenocarcinoma to a lymph node. Specifically, a tissue section of lung of a patient with lymph node metastasis and a tissue section of lung of a patient without lymph node metastasis are prepared as samples, and a molecule useful as a metastasis diagnosis marker is identified by measuring an expression amount of a variety of molecules. In addition, there is the disclosure that, by using this molecule, it is possible to diagnose metastasis of a lung cancer to a lymph node in a patient with pulmonary adenocarcinoma. However, this technique can not determine to what an extent a cancer has been metastasized to a lymph node.

Methods and apparatuses for the amplification of nucleic acids, including e.g. the amplification of CK19 mRNA are known e.g. from EP 1 813 682. Such methods and apparatuses are disclosed in the specific context of metastasis of malignant tumor, e.g. in EP 1 867 735.

### SUMMARY

The scope of the present invention is defined solely by the appended claims, and is not affected to any degree by the statements within this summary.

An object of the present invention is to provide a method which can objectively determine to what an extent a cancer has been metastasized to a lymph node.

The present invention provides a method for determining a size of a metastatic focus, comprising a step of quantitating a cytokeratin 19 mRNA in an assay sample prepared from a lymph node suspected of metastasis of a cancer cell, and a step of obtaining a size of a metastatic focus in the lymph node based on the quantitation result of the mRNA.

Also, the present invention provides an apparatus for determining cancer metastasis according to the provided method, comprising a quantitation part for quantitating cytokeratin 19 mRNA in an assay sample prepared from a lymph node suspected of metastasis of a cancer cell, a comparison part for comparing the quantitation result of the mRNA with a first threshold and a second threshold which is higher than the first threshold, and a determination part for determining whether cancer metastasis of the lymph node is negative, weakly positive or strongly positive, based on the comparison result.

According to the present invention, to what an extent a cancer cell has been metastasized to a lymph node can be determined.

### BRIEF DESCRIPTION OF THE DRAWINGS

Fig. 1 is a perspective view showing a whole construction of an apparatus 1 in accordance with one embodiment of the present invention;
Fig. 2 is a perspective view showing a whole construction of a nucleic acid quantitation part 101;
Fig. 3 is a schematic plane view of the nucleic acid quantitation part 101;
Fig. 4 is a block view showing a construction of a control part 102d;
Fig. 5 is a process flow with the control part 102d;
Fig. 6 is one example of a screen for displaying the determination result in the display part 102c;
Fig. 7 is a graph showing a relationship between a size of a metastatic focus of a breast cancer and the quantitation result of a CK19mRNA;
Fig. 8 is a graph showing a relationship between a size of a metastatic focus of a large intestine cancer and the quantitation result of a CK19mRNA;
Fig. 9 is a graph showing a relationship between an expression amount of an mRNA in a lymph node to which a cancer cell derived from papillotubular carcinoma has been metastasized, and the number of cancer cells contained in a metastatic focus;
Fig. 10 is a graph showing a relationship between an expression amount of an mRNA in a lymph node to which a cancer cell derived from solid tubular carcinoma has been metastasized, and the number of cancer cells contained in a metastatic focus;
Fig. 11 is a presumed value of an expression amount of a CK19mRNA in a lymph node having a metastatic focus of a particular volume;
Fig. 12 is a graph showing the quantitation result of a CK19mRNA when a lymph node taken from a breast cancer patient is used; and
Fig. 13 is a graph showing the quantitation result of a CK19mRNA when a lymph node taken from a large intestine cancer patient is used.

### DETAILED DESCRIPTION OF THE INVENTION

One embodiment of the present invention is a method for determining cancer metastasis contained in a lymph node taken from a living body. According to this method, a size of a metastatic focus of a cancer in a lymph node (hereinafter, also simply referred to as metastatic focus) can be obtained.

Herein, a size of a metastatic focus is a concept including a major axis and an area of a metastatic focus prepared upon preparation of a tissue section from a lymph node, a volume of a metastatic focus calculated from this major axis, a mass of a metastatic focus, and the number of cancer cells contained in a metastatic focus.

The cancer as used herein is a tumor which has become malignant, and has the same meaning as that of a malignant tumor. The cancer includes carcinoma, sarcoma, and a cancer derived from a hematopoietic organ. Examples of the carcinoma include a cancer derived from an epithelial cell such as a breast cancer, a stomach cancer, a large intestine cancer, a prostate cancer, a cervical cancer, and a cancer of uterine body. Examples of the sarcoma include osteosarcoma and soft-tissue sarcoma. Examples of the cancer derived from a hematopoietic organ include leukemia and malignant lymphoma.

In order to obtain a size of a metastatic focus, first, a tumor marker mRNA (hereinafter, also simply referred to as mRNA) contained in a lymph node is quantitated. The tumor marker mRNA is an mRNA transcribed from a tumor marker gene. The tumor marker gene refers to such a gene that an expression amount in a cancer cell and an expression amount in a normal cell are significantly different as described above. Examples of the tumor marker gene include genes encoding CKs (cytokeratins) such as CK18, CK19 and CK20, CEA (carcinoembryonic antigen), MUC1, MMG (mammaglobin), PSA (prostate specific antigen), CA15-3, EpCAM (epithelial cellular adhesion molecule) and the like.

Upon quantitation of an mRNA, it is preferable that an assay sample is prepared from the lymph node, and an mRNA contained in this assay sample is quantitated. For example, a lymph node and a buffer are mixed, a cell in the buffer is chemically and/or physically treated to transfer an RNA in a cell into a solution (solubilization), and a solution containing this RNA can be used as an assay sample.

In order to suppress degradation of an RNA, it is preferable that the buffer is strongly acidic. A range of a pH is preferably 2.5 to 5.0, more preferably 3.0 to 4.0. In order to keep a pH in this range, known buffers can be used.

In addition, it is preferable that a surfactant is contained in the buffer. The surfactant damages a cell membrane or a nuclear membrane. Through this damage, it becomes easy for a nucleic acid in a cell to transfer into a solution. A kind of the surfactant is not particularly limited as far as it has such action, but a nonionic surfactant is preferable, and a polyoxyethylene-based nonionic surfactant is more preferable.

In particular, a polyoxyethylene-based nonionic surfactant represented by the following general formula:

R1-R2-(CH₂CH₂O)n-H

(wherein R1 is an alkyl group, an alkenyl group, an alkynyl group or an isooctyl group having 10 to 22 carbon atoms, or; R2 is -O- or -(C₆H₆)-O-; n is an integer of 8 to 120) is suitable. Examples thereof include polyoxyethylene lauryl ether, polyoxyethylene cetyl ether, polyoxyethylene oleyl ether, polyoxyethylene myristyl ether, polyoxyethylene strearyl ether, polyoxyethylene nonyl phenyl ether, and polyoxyethylene isooctyl phenyl ether. Specifically, Brij 35 (polyoxyethylene(35)lauryl ether) is suitable. A concentration of the surfactant in the buffer is preferably 0.1 to 6% (v/v), more preferably 1 to 5% (v/v).

In addition, when quantitation of an mRNA is performed by nucleic acid amplification described later, it is preferable that dimethyl sulfoxide (DMSO) is contained in the buffer. A substance which inhibits an enzymatic reaction in nucleic acid amplification (inhibitory substance) is contained in a lymph node in some cases, and an influence of this inhibitory substance can be effectively reduced by the action of DMSO. In addition, DMSO also has the effect of reducing the activity of a nucleic acid amplification enzyme. A concentration of DMSO in a treating solution is preferably 1 to 50% (v/v), more preferably 5 to 30% (v/v), most preferably 10 to 25% (v/v).

Upon preparation of an assay sample, it is preferable that a lymph node is subjected to physical treatment such as homogenization. Thereby, a cell membrane or a nuclear membrane of a cell in a lymph node is physically fragmented, and it becomes easy for a nucleic acid in a cell to transfer into a solution. Homogenization may be performed manually with a pestle or the like, or may be performed using a commercially available electric homogenizer. Debris of a cell floating in the resulting homogenate can be precipitated by centrifuging the homogenate for a few seconds to a few minutes. Thereby, a supernatant containing an RNA and the like (lysate) can be prepared as an assay sample.

Quantitation of an mRNA can be performed by a known method using nucleic acid amplification, a DNA chip or the like. When the nucleic acid amplification method is used, an RT-PCR (Reverse Transcription PCR) method and an RT-LAMP method (Reverse Transcription LAMP: for the LAMP method, see US6410278) comprising a reverse transcription reaction before a nucleic acid amplification reaction are suitably used. In particular, as a quantitative nucleic acid amplification method, known methods such as a Green method and a TaqMan (registered trademark of Roche Diagnostic) method (see Linda G. et al., 1993, Nucleic Acids Research, vol. 21, p. 3761-3766 etc.) can be used.

As a DNA chip which can be used in quantitation of an mRNA, a substrate on which a polynucleotide and/or a fragment thereof of a DNA being capable of hybridizing with a cDNA of the tumor marker gene is immobilized can be used. Detection of an RNA using the DNA chip can be performed by generally used known methods. For example, detection can be performed as follows. First, utilizing a poly A sequence present at a 3'-terminus of an mRNA in an assay sample, a reverse transcription reaction is performed. By using a nucleotide labeled with a fluorescent substance such as Cy3 and Cy5 upon a reverse transcription reaction, a fluorescently labeled cDNA is synthesized. When this is contacted with the substrate on which the polynucleotide is immobilized, this polynucleotide and the labeled cDNA form a double strand. After formation of the double strand, fluorescence of the cDNA can be measured to quantitate an mRNA.

The quantitation result of an mRNA measured by the above method may be a substance amount of an mRNA, an mRNA mass, or a copy number per unit area. Alternatively, the result may be a time or a cycle number (in the case of using PCR) until a fluorescence intensity, a turbidity or a transmitted light intensity of the reaction solution become predetermined values.

Usually, an mRNA of a gene thought to be constitutively expressed in any cell (e.g. housekeeping gene etc. : hereinafter, referred to normalized gene) is quantitated together with quantitation of the tumor marker mRNA, and a quantitated value of the tumor marker mRNA is divided (normalized) by a quantitated value of an mRNA of a normalized gene or the like, thereby, it is converted into an expression amount of the tumor marker mRNA per mRNA of the normalized gene (e.g. see M. Inokuchi et al., British Journal of Cancer (2003) 89, 1750-1756).

However, when not only a cancer cell but also a normal lymph node cell is contained in a lymph node in a large amount, since the measurement result is influenced by the number of normal cells, a size of a metastatic focus is not correctly obtained in some cases. Therefore, in the present embodiment, it is preferable that normalization is not performed to the quantitated value of the tumor marker mRNA, and the quantitated value (absolute value) is used as it is.

The mRNA quantitation result is correlated with a size of a metastatic focus. For this reason, a size of a metastatic focus in a lymph node can be obtained based on the mRNA quantitation result as described above. Alternatively, a size of a metastatic focus may be obtained stepwise. When a stepwise size of a metastatic focus is obtained, the mRNA quantitation result and a plurality of thresholds are compared. In this case, it is preferable that at lease one threshold (first threshold) is set so that cancer negative and cancer positive can be discriminated.

The threshold is appropriately set depending upon a kind of a cancer or the tumor marker. In addition, when a size of a metastatic focus is obtained, it is preferable that this first threshold is used to discriminate negative and positive, and a size of a metastatic focus is obtained regarding metastasis positive.

The first threshold can be set at a value not higher than a quantitated value of an mRNA contained in a lymph node for which the presence of a cancer cell has been confirmed (positive specimen), and higher than a quantitated value of an mRNA contained in a lymph node for which the absence of a cancer cell has been confirmed (negative specimen). It is preferable that mRNA quantitated values of a plurality of positive specimens and mRNA quantitated values of a plurality of negative specimens are measured in advance, and a value by which a positive specimen and a negative specimen can be discriminated at the highest probability is set as a threshold.

When the aforementioned stepwise information is obtained, a second threshold is further used in addition to the first threshold. That is, the second threshold by which weakly positive and strongly positive can be discriminated is used in addition to the first threshold. Thereby, when the quantitation result of an mRNA is less than the first threshold, it can be determined that a metastatic focus is not substantially present (i.e. cancer metastasis negative). In addition, when the quantitation result of an mRNA is not less than the first threshold and less than the second threshold, it can be determined that a relatively small metastatic focus is present (cancer metastasis weakly positive). Further, when the quantitation result of an mRNA is not less than the second threshold, it can be determined that a relatively large metastatic focus is present (cancer metastasis strongly positive).

A metastatic focus in a lymph node can be classified into micro metastasis and macro metastasis based on a size. Metastasis in which a metastatic focus is confirmed and the metastatic focus has a major axis of less than 2 mm in a tissue section prepared from a lymph node tissue is called micro metastasis. Metastasis in which the metastatic focus has a major axis of not less than 2 mm is called macro metastasis. The second threshold can be set at a value by which micro metastasis and macro metastasis can be discriminated. Alternatively, a plurality of thresholds may be set depending upon a size of a metastatic focus, instead of the second threshold.

In addition, quantitative information of a size of a metastatic focus can also be obtained from an expression amount of a tumor marker mRNA. That is, the number of cancer cells in a lymph node, an area, a volume or a mass of a metastatic focus, and the like can be obtained quantitatively.

Another embodiment of the present invention is an apparatus for determining cancer metastasis for carrying out the aforementioned method. When this apparatus is used, a size of a metastatic focus in a lymph node can also be stepwisely obtained. This apparatus will be explained below referring to the drawings.

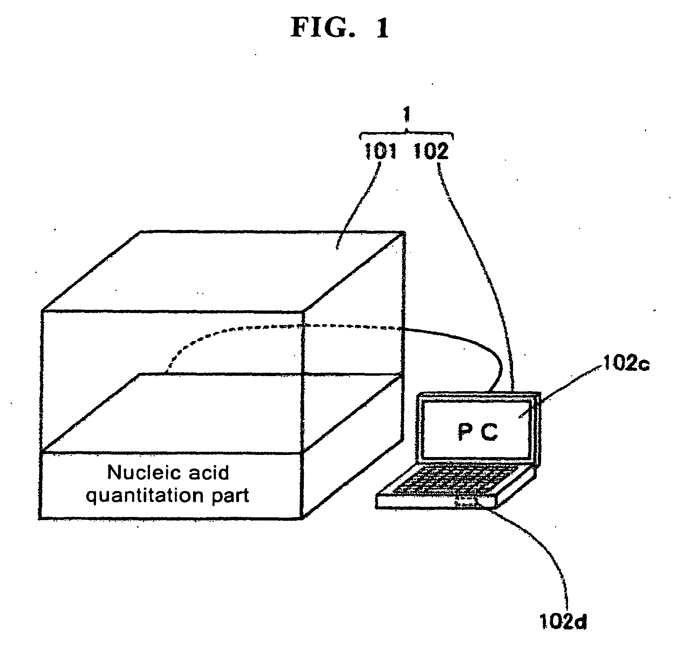

Fig. 1 is a perspective view showing a whole construction of an apparatus 1 in accordance with one embodiment of the present invention. This apparatus 1 is constructed of a nucleic acid quantitation part 101, and a personal computer (PC) 102 which is connected so as to communicate with the nucleic acid quantitation part 101 by a wire. The personal computer 102, as shown in Fig. 1, comprises an inputting part 102 including a keyboard, a display part 102c including a monitor, and a control part 102d for analyzing the measurement result.

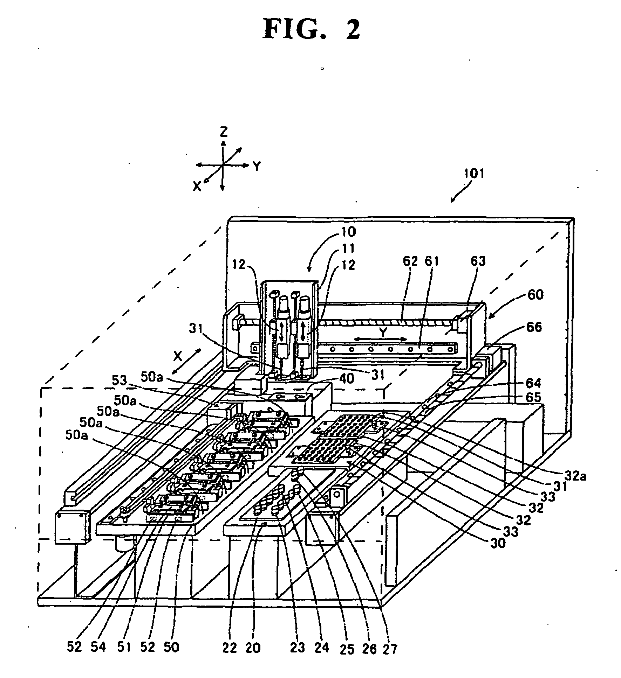

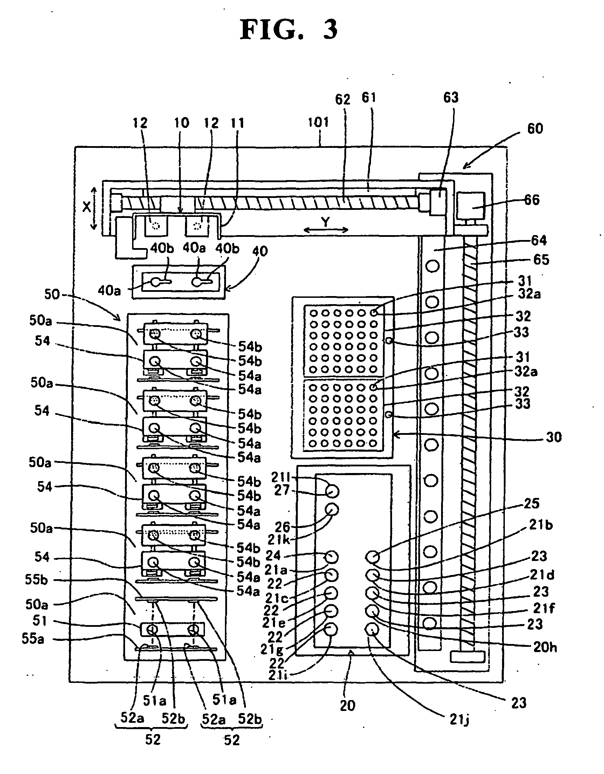

The nucleic acid quantitation part 101 of the apparatus 1 will be explained below using Fig. 2 and Fig. 3. Fig. 2 is a perspective view showing a whole construction of the nucleic acid quantitation part 101. Fig. 3 is a schematic plane view of the nucleic acid quantitation part 101 of Fig. 2.

The nucleic acid quantitation part 101, as shown in Fig. 2, comprises a dispensing mechanism 10, a sample setting part 20, a tip setting part 30, a tip disposal unit 40, a reaction detection part 50 consisting of five reaction detection blocks 50a, and a transfer part 60 for transferring the dispensing mechanism 10 in an X axis direction and a Y axis direction.

In addition, the dispensing mechanism 10, as shown in Fig. 2, comprises an arm part 11 which is moved in an X axis direction and a Y axis direction (horizontal direction) by the transfer part 60, and duplicate (two) syringe units 12 which can be independently moved in a z axis direction (vertical direction) with respect to the arm part 11.

In addition, as shown in Fig. 2 and Fig. 3, ten sample container holes 21a to 21j, one enzyme reagent container hole 21k and one primer reagent container hole 211 are provided in the sample setting part 20 in an order from the front of the apparatus. In addition, ten sample container holes 21a to 21j are provided so as to be arranged in 5 rows and 2 columns. In addition, the sample container holes 21c and 21d, the sample container holes 21e and 21f, the sample container holes 21g and 21h, and the sample container holes 21i and 21j are provided at a sample position 1, a sample position 2, a sample position 3 and a sample position 4, respectively, from an inner side of the apparatus.

In addition, in the present embodiment, a sample container 22 accommodating a lysate (assay sample) prepared by subjecting a pre-excised biological tissue to the aforementioned treatment (homogenization, filtration) is set in the sample container holes 21c, 21e, 21g and 21i on a front left side and, at the same time, a sample container 23 accommodating a diluted sample obtained by 10-fold diluting the aforementioned sample is set in the sample container holes 21d, 21f, 21h and 21j on a front right side.

In addition, a container 24 accommodating a positive control (InCt) for confirming that a nucleic acid to be amplified is normally amplified is mounted in the sample container hole 21a and, at the sample time, a container 25 accommodating a negative control for confirming that a nucleic acid not to be amplified is not normally amplified is set in the sample container hole 21b.

In addition, an enzyme reagent container 26 accommodating a nucleic acid amplification enzyme reagent for amplifying a cDNA (hereinafter, also referred to as CK19cDNA) corresponding to a CK19 mRNA (tumor marker mRNA: hereinafter, also referred to as CK19mRNA), and a primer reagent container 27 accommodating a reagent (hereinafter, referred to as primer reagent) containing a primer which can hybridize with the CK19cDNA are set in the enzyme reagent container hole 21h and the primer reagent container hole 211, respectively.

In addition, each reaction detection block 50a of the reaction detection part 50, as shown in Fig. 2 and Fig. 3, is constructed of a reaction part 51, two turbidity detection parts 52, and a lid closing mechanism 53 (see Fig. 3). Two detection cell holes 51a for setting a detection cell 54 are provided in a reaction part 51 provided in each reaction detection block 50a, as shown in Fig. 3. Respective reaction detection blocks 50a are arranged at a cell position 1, a cell position 2, a cell position 3, a cell position 4 and a cell position 5 in an order from an inner side of the apparatus.

In addition, the turbidity detection part 52 is constructed of an LED light source part 52a consisting of a blue LED having a wavelength of 456 nm attached to a substrate 55a arranged on one side surface of the reaction part 51, and a photodiode light receiving part 52b attached to a substrate 55b arranged on the other side surface of the reaction part 51. Two sets of turbidity detection parts 52, one set consisting of one. LED light source part 52a and one photodiode light receiving part 52b, are arranged in each reaction detection block 50a.

In addition, the detection cell 54 has two cell parts 54a for accommodating a sample, and two lid parts 54b for closing the two cell parts 54a.

In addition, the transfer part 60, as shown in Fig. 2, comprises a direct acting guide 61 and a ball screw 62 for transferring the dispensing mechanism 10 in a Y axis direction, a stepping motor 63 for driving the ball screw 62, a direct acting guide 64 and a ball screw 65 for transferring the dispensing mechanism 10 in an X axis direction, and a stepping motor 66 for driving the ball screw 65. Transference of the dispensing mechanism 10 in the X axis direction and the Y axis direction is performed by rotating the ball screw 62 and the ball screw 65, respectively, with the stepping motors 63 and 66.

Then, referring to Fig. 1 to Fig. 3, motion of the nucleic acid quantitation part 101 in accordance with the present embodiment will be explained. In this embodiment, as described above, a cDNA corresponding to a CK19mRNA (tumor marker) in a lymph node tissue excised by an operation is amplified using the RT-LAMP method, and a change in a turbidity due to clouding of magnesium pyrophosphate generated accompanied with amplification is measured. Based on a time until the turbidity reaches a predetermined value (amplification starting time), an amount of the CK19mRNA (copy number/µL • lysate) is measured, and this amount is compared with a threshold.

First, as shown in Fig. 2 and Fig. 3, the sample container 22 accommodating an assay sample prepared by subjecting an excised tissue to treatment (homogenization, filtration) in advance is set in the sample container holes 21c to 21j. In addition, the container 24 accommodating a positive control and the container 25 accommodating a negative control are set in the sample container holes 21a and 21b, respectively (see Fig. 3). In addition, the enzyme reagent container 26 accommodating a nucleic acid amplification enzyme reagent for amplifying the CK19cDNA, and the primer reagent container 27 accommodating a primer reagent for amplifying the CK19cDNA are set in the enzyme reagent container hole 21k (see Fig. 3) and the primer reagent container hole 211, respectively. In addition, two racks 32 accommodating thirty six disposable pipette tips 31 are mounted in a tip setting part 30.

When motion of the nucleic acid quantitation part 101 is started, first, after the arm part 11 of the dispensing mechanism 10 is transferred from an initial position to the tip setting part 30 by the transfer part 60 shown in Fig. 2, the two syringe units 12 of the dispensing mechanism 10 are moved downwardly in the tip setting part 30. Thereby, since tips of nozzle parts of the two syringe units 12 are pressed into upper openings of the two pipette tips 31, pipette tips 31 are automatically mounted on tips of nozzle parts of the two syringe units 12. Then, after the two syringe units 12 are moved upwardly, the arm part 11 of the dispensing mechanism 10 is moved in an X axis direction towards above the primer reagent container 27 accommodating a primer reagent. Then, after one syringe unit 12 situated at above the primer reagent container 27 is moved downwardly, and the primer reagent is sucked, the one syringe unit 12 is moved upwardly. Thereafter, the arm part 11 of the dispensing mechanism 10 is moved in a Y axis direction by the transfer part 60 so that the other syringe unit 12 is situated at above the same primer reagent container 27. Then, after the other syringe unit 12 is moved downwardly, and a primer reagent is sucked from the same primer reagent container 27, the other syringe unit 12 is moved upwardly. In such a way, the primer reagent in the primer reagent container 27 is sucked by two pipette tips 31 mounted on the syringe units 12.

After suction of the primer reagent and after the two syringe units 12 are moved upwardly, the arm part 11 of the dispensing mechanism 10 is moved by the transfer part 60 towards above the reaction detection block 50a situated at the cell position 1 which is on an innermost side (inner side in front of apparatus). In addition, in the reaction detection block 50a which is on an innermost side, the two pipette tips 31 mounted on the two syringe units 12 are inserted into the two cell parts 54a of the detection cell 54, respectively, by moving the two syringe units 12 downwardly. In addition, primer reagents are discharged into the two cell parts 54a using the syringe units 12.

After discharge of the primer reagent and after the two syringe units 12 are moved upwardly, the arm part 11 of the dispensing mechanism 10 is moved by the transfer part 60 in an X axis direction towards above the tip disposal unit 40. Then, in the tip disposal unit 40, the pipette tip 31 is discarded. Specifically, by moving the two syringe units 12 downwardly, the pipette tip 31 is inserted into the two tip disposal holes 40a (see Fig. 3) of the tip disposal unit 40. In this state, by moving the arm part 11 of the dispensing mechanism 10 in a Y axis direction with the transfer part 60, the pipette tip 31 is moved towards below a groove part 40b. In addition, by moving the two syringe units 12 upwardly, since a collar part on an upper side of the pipette tip 31 is abutted against undersides on both sides of the groove part 40b, and undergoes a downward force from the undersides, the pipette tip 31 is automatically detached from nozzle parts of the two syringe units 12. Thereby, the pipette tip 31 is discarded into the tip disposal unit 40.

Then, by a similar motion, the enzyme reagent is discharged from the enzyme reagent container 26 into the cell part 54a and, further by a similar motion, a sample is discharged from the sample container 22 and the sample container 23 into the cell part 54a.

Then, after discharge of the primer reagent, the enzyme reagent and the sample into the cell part 54a, lid closing motion of the lid part 54b of the detection cell 54 is performed. After this lid closing motion is completed, a liquid temperature in the detection cell 54 is raised from about 20°C to about 65°C, thereby, a cDNA corresponding to the CK19mRNA is amplified by the RT-LAMP reaction. Then, clouding due to magnesium pyrophosphate generated accompanied with amplification is detected by nephelometry. Specifically, using the LED light source part 52a and the photodiode light receiving part 52b shown in Fig. 3, a turbidity in the detection cell 54 at the time of an amplification reaction is detected (monitored), thereby, a turbidity is detected.

Turbidity data of the sample is sent from the nucleic acid quantitation part 101 to the control part 102d of the personal computer 102 in real time.

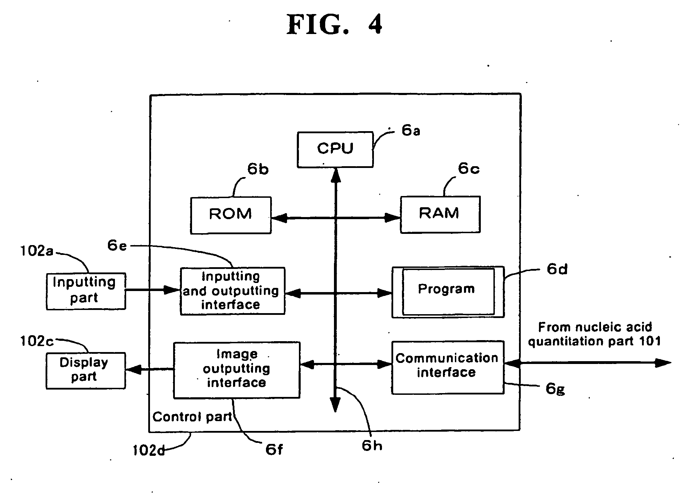

Fig. 4 is a block view showing a construction of the control part 102d. This control part 102d is constructedmainly of a CPU 6a, an ROM 6b, an RAM 6c, a hard disk 6d, an inputting and outputting interface 6e, an image outputting interface 6f, and a communication interface 6g, and the CPU 6a, the ROM 6b, the RAM 6c, the hard disk 6d, the inputting and outputting interface 6e, the image outputting interface 6f, and the communicating interface 6g are data-communicatively connected with a bus 6h.

The CPU 6a can execute a computer program memorized in the ROM 6b and the hard disk 6d, and a computer program read out into the RAM 6c.

The ROM 6b memorizes a computer program which is executed by the CPU 6a, data used for executing the computer program, and the like.

The RAM 6c is used as a working area when a computer program memorized in the ROM 6b and the hard disk 6d is read out, or when a computer program is executed.

The hard disk 6d memorizes a computer program to be executed by the CPU 6a, and data to be used for executing the computer program. This computer program fulfills a function of analyzing turbidity information sent from the nucleic acid quantitation part 101 and outputting the analysis result.

The inputting part 102a including a keyboard is connected to the imputing and outputting interface 6e. The inputting part 102a is provided for an operation on an outputted screen, and the like. The image outputting interface 6f is connected to the display part 102c. The display part 102c is provided for displaying a turbidity sent from the nucleic acid quantitation part 101 in real time, and outputting the analysis result. The communication interface 6g is connected to the nucleic acid quantitation part 101, and fulfills a function of receiving turbidity information.

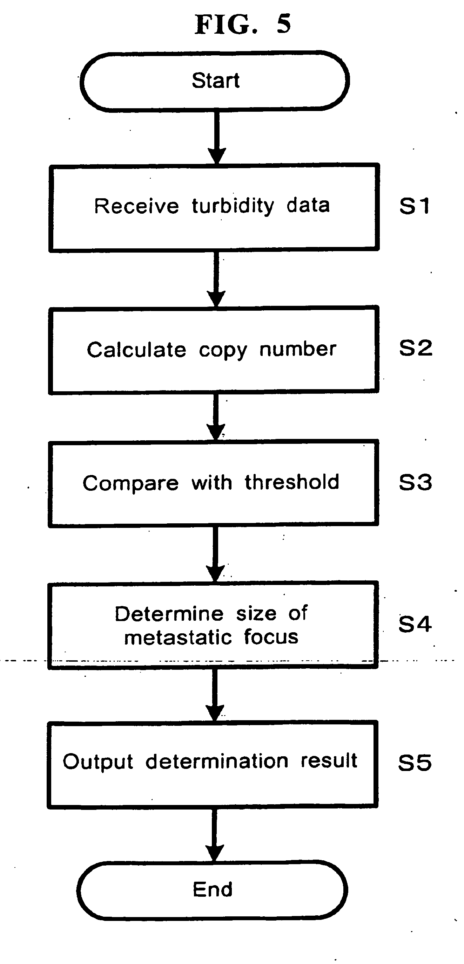

Then, referring to Fig. 5, a process flow using the control part 102d will be explained. The control part 102d receives turbidity data from the nucleic acid quantitation part 101 in real time (step S1). A copy number (copy/uL • lysate) of an mRNA is calculated from a time at which a turbidity of a reaction solution reaches a predetermined value (step S2), and this number is compared with a predetermined threshold (first threshold and second threshold), thereby, a size of a metastatic focus is determined (step S3). Specifically, when the mRNA copy number is less than 250 copies/µL • lysate (first threshold), it is determined that cancer metastasis is negative, that is, a metastatic focus is not substantially present. When the mRNA copy number is not less than 250 copies/µL • lysate (first threshold) and less than 5000 copies/µL • lysate (second threshold), it is determined that cancer metastasis is weakly positive (+), that is, micro metastasis is present. When the mRNA copy number is more than 10000 copies/µL •lysate (second threshold), it is determined that cancer metastasis is strongly positive (++), that is, macro metastasis is present. Then, the control part 102d outputs these determination results into the display part 102c of the personal computer 102 (step S4) to make the display part 102c display the determination results.

The " copy/µL • lysate" indicates the mRNA copy number in 1 µL of a lysate obtained by solubilizing a lymph node tissue having a diameter of about 6 mm in 4 mL of a buffer (pH 3.4: containing 200 mM glycine HCl, 5% Brij 35 (polyoxyethylene(35)lauryl ether, manufactured by Sigma) and 20% DMSO (Wako Pure Chemical Industries, Ltd.)), and further 10-fold diluting the solubilized product.

In the above embodiment, the first threshold is set preferably between 50 to 3000 copies/µL • lysate, more preferably between 100 to 1000 copies/ µL • lysate, further preferably between 200 to 300 copies/ µL • lysate. In addition, the second threshold is set preferably between 2000 to 20000 copies/µL • lysate, further preferably between 4000 to 10000 copies/µL • lysate.

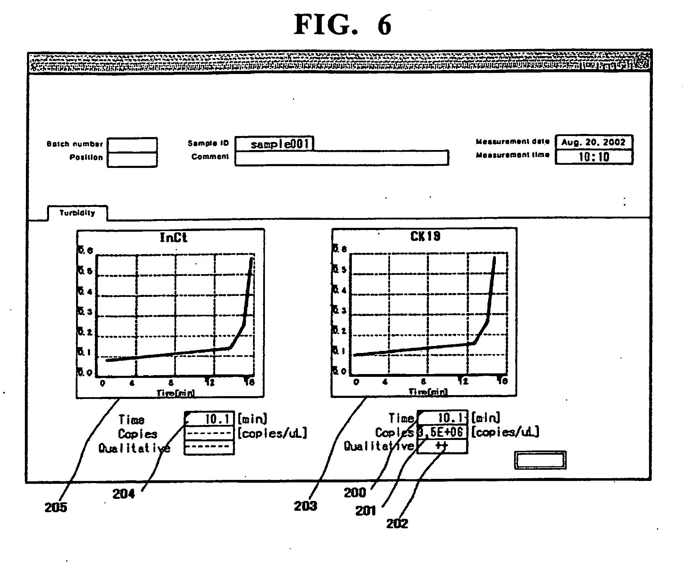

Then, referring to Fig. 6, one example of a screen for displaying the determination result in the display part 102c will be explained. An amplification starting time for the CK19mRNA is displayed in a column 200, and an amplification starting time for a positive control is displayed in a column 205. A CK19mRNA expression amount (copy/µL) calculated by the CPU 6a based thereon is displayed in a column 201. Information regarding a size of a metastatic focus obtained by comparing this expression amount with a first threshold and a second threshold is displayed in a column 202. In Fig. 6, ++, that is, the fact of cancer strongly positive is shown. In addition, a real time curve of a turbidity of a reaction solution in which the CK19mRNA was amplified is displayed in a column 203, and a real time curve of a turbidity of a reaction solution in which the positive control was amplified is displayed in a column 205.

In the apparatus of the above embodiment, based on the result of comparison with thresholds, a size of a metastatic focus in a lymph node was stepwisely determined. However, as described above, an area of a metastatic focus, and the number of cells contained in a metastatic focus may be quantitatively obtained from the result of calculation of the mRNA copy number.

### EXAMPLES

### (Example 1)

### (1) Immunohistochemistry

Using 11 lymph node tissues excised from breast cancer patients, and 7 lymph nodes excised from large intestine cancer patients, an area of a metastatic focus (metastatic focus size) was measured by immunohistochemistry.

At a place near a center of each of these lymph nodes (about 50 to 600 mg/node), a section having a thickness of about 10 µm (hereinafter, also referred to as section) was excised, and placed on a glass slide. For this section, a cancer cell was stained using an anti-CK19 antibody and an Envision Kit (both DAKO). Using a GS-710 Calibrated Densitometer (Bio-rad), a metastatic focus size of a cancer cell of this section was measured. The measurement results (mm²) are shown in the following Table 1.

### (2) Quantitation of CK19mRNA

A section (thickness about 10 µm) adjacent to the above-excised section was excised, and an RNA was extracted using an RNeasy Mini Kit (Qiagen) to prepare an RNA sample. Using this RNA sample, a reaction solution having the following composition was prepared, and a copy number of the CK19mRNA was calculated by a TaqMan method. Measurement by the TaqMan method was performed according to an attached use instruction using a TaqMan One-step RT-PCR Master Mix and a Prism 7700 Realtime PCR system (both Applied Biosystems).
Composition of reaction solution (50 µL)
Purified water 20.10 µL
RNA sample 2 µL
TaqMan 2 × Universal PCR Master Mix 25 µL
40 x MultiScribe and RNase Inhibitor Mix 1.25 µL
100 µM forward primer 0.15 µL
100 µM reverse primer 0.15 µL
7.4 pmol/ µL (final concentration 200 nm) TaqMan probe 1.35 µL

In addition, a primer sequence and a sequence of the TaqMan probe for detecting the CK19mRNA are as follows.
Forward primer: 5'-CAGATCGAAGGCCTGAAGGA-3' (SEQ ID No.: 1)
Reverse primer: 5'-CTTGGCCCCTCAGCGTACT-3' (SEQ ID No.: 2)
TaqMan probe: 5'-GCCTACCTGAAGAAGAACCATGAGGAGGAA-3' (SEQ ID No.: 3)

In addition, this TaqMan probe has 6-carboxyfluorescein (FAM) at a 5'-terminus, and 6-carboxy-tetramethyl-rhodamine (TAMRA) at a 3'-terminus.

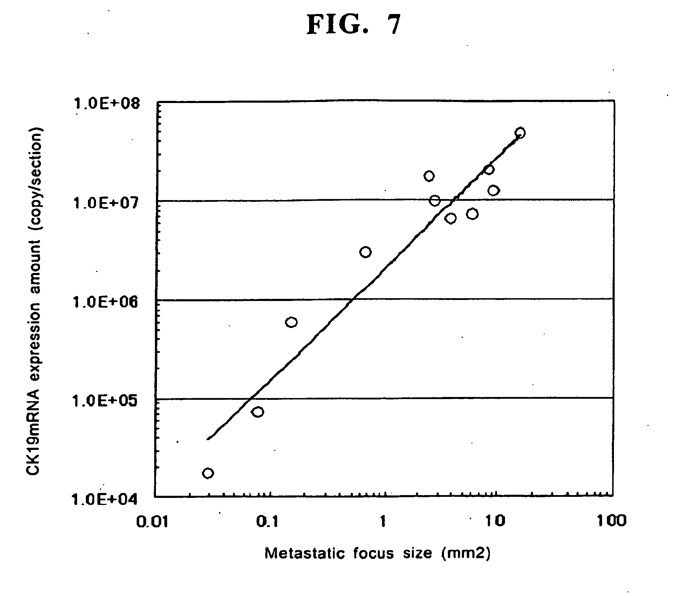

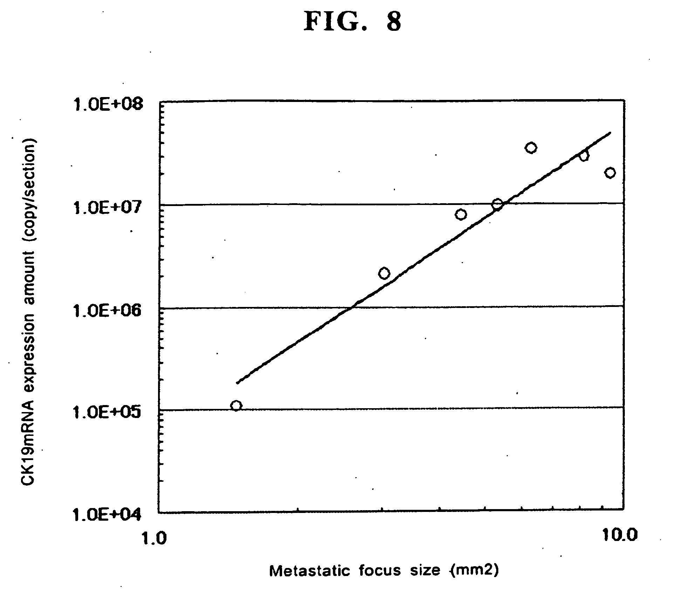

Quantitation values (copy/section) of an mRNA are shown in the following Table 1. In addition, a relationship between a metastatic focus size of a breast cancer and the CK19mRNA quantitation result is shown in Fig. 7, and a relationship between a metastatic focus size of a large intestine cancer and the CK19mRNA quantitation result is shown in Fig. 8.

**Table 1**

| | Size of metastatic focus (mm2) | CK19mRNA (copy/section) |
|---|---|---|
| Breast cancer | 0.67 | 3.00E + 06 |
| | 0.029 | 1.07E + 04 |
| | 2.82 | 9.69E + 06 |
| | 8.4 | 2.03E + 07 |
| | 9.36 | 1.23E + 07 |
| | 6.14 | 7.20E + 06 |
| | 2.51 | 1.70E + 07 |
| | 0.079 | 7.20E + 04 |
| | 0.15 | 5.08E + 05 |
| | 15.68 | 4.70E + 07 |
| | 3.91 | 6.40E + 06 |
| Large intestine cancer | 4.45 | 8.00E + 06 |
| | 9.39 | 2.00E + 07 |
| | 8.18 | 2.90E + 07 |
| | 6.29 | 3.40E + 07 |
| | 5.33 | 9.70E + 06 |
| | 1.47 | 1.10E + 05 |
| | 3.04 | 2.10E + 06 |

From Table 1, and Figs. 7 and 8, either in a large intestine cancer or a breast cancer, a correlation relationship was recognized between the quantitation result of the CK19mRNA and a size (mm²) of a metastatic focus. Therefore, it is confirmed that a size (mm²) of a metastatic focus can be predicted by quantitating the CK19mRNA.

### (Example 2)

Thirty five sections having a thickness of about 10 µm were excised from one lymph node to which a cancer cell derived from papillotubular carcinoma had been metastasized at a constant interval. The thirty five sections were subjected to immunohistochemistry as in Example 1(1), and the number of cancer cells (number/section) was counted.

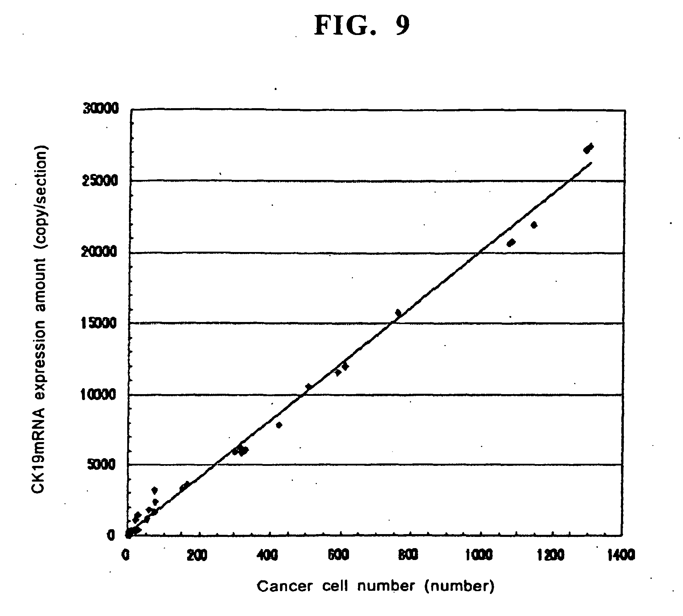

Since the section used in immunohistochemistry can not be used in quantitation of an mRNA, an expression amount of an mRNA (copy/section) of a section (thickness about 10 µm) adjacent to this section was measured as in Example 1, and this quantitation result was made to correspond to the number of cancer cells with immunohistochemistry. A relationship between the mRNA expression amount and the cancer cell number is shown in Fig. 9.

Thirty sections having a thickness of about 10 µm were excised from one lymph node to which a cancer cell derived from solid tubular carcinoma had been metastasized, at a constant interval. The thirty sections were subjected to immunohistochemistry as in Example 1(1), and the number (number/section) of cancer cells was counted. Then, using a section adjacent to each section, an expression amount (copy/section) of an mRNA was measured as in Example 1(2).

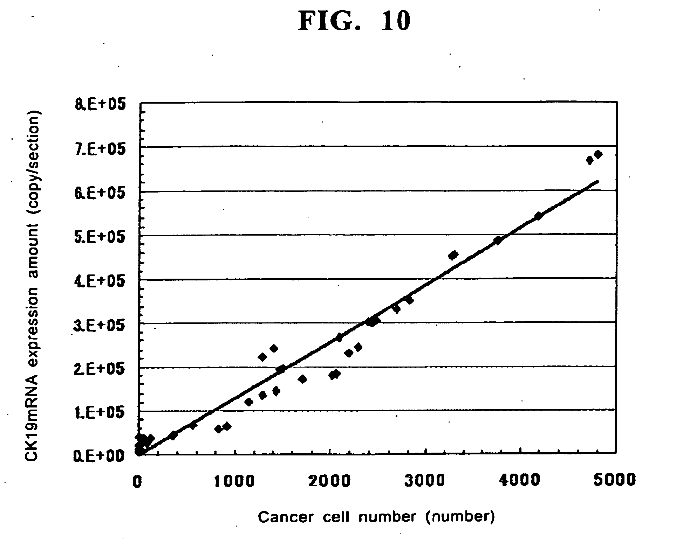

In the same manner as that of Example 2, a relationship between the mRNA expression amount and the cancer cell number is shown in Fig. 10, letting the mRNA expression amount in a predetermined section which was subjected to cancer cell counting to be the mRNA expression amount of an adjacent section.

As shown in Figs. 9 and 10, a good correlation relationship was shown between the expression amount of the CK19mRNA and the cancer cell number. Therefore, it was confirmed that the number of cancer cells in a metastatic focus can be predicted by quantitating the CK19mRNA.

### (Example 3)

Using eleven lymph nodes for which cancer metastasis was not recognized (negative specimen) and in the same manner as that of Example 1(2), an assay sample was prepared, and an expression amount of the CK19mRNA (background value) in each specimen was measured.

In addition, based on data on a breast cancer shown in Table 1, an expression amount of a CK19mRNA when a metastatic focus in each section was postulated to be a cube having 1 mm on one side (volume 1 mm³), and an expression amount of a CK19mRNA when the metastatic focus was postulated to be a cube having 2 mm on one side (volume 8 mm³) were calculated.

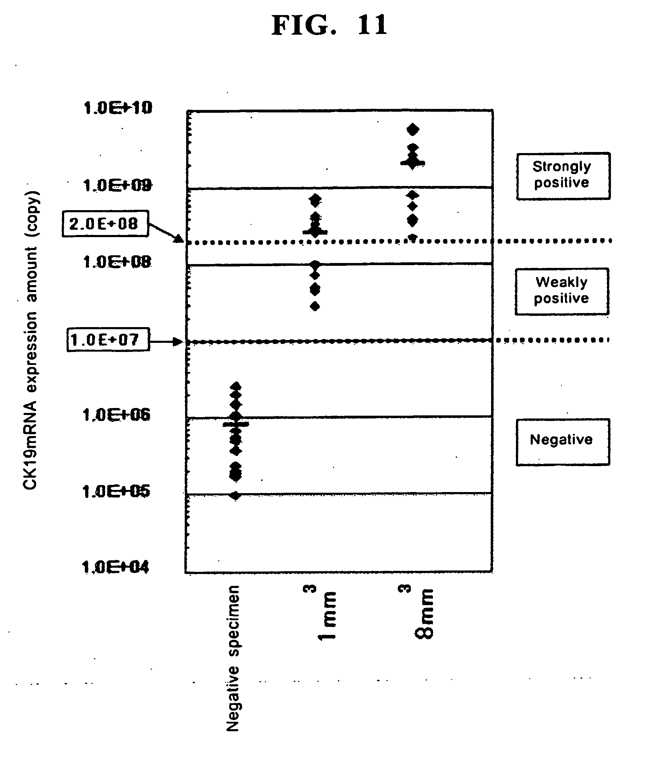

Further, an expression amount of a CK19mRNA of a negative specimen which is a background value was added to each of an expression amount of a CK19mRNA when a metastatic focus was 1 mm³, and an expression amount of a CK19mRNA when the metastatic focus was 8 mm³. That is, these values are an expression amount of a CK19mRNA (background) of one lymph node having no metastatic focus, a presumed value of an expression amount of a CK19mRNA of one lymph node having a metastatic focus of 1 mm³, and a presumed value of an expression amount of a CK19mRNA of one lymph node having a metastatic focus of 8 mm³. Theses values are shown in Fig. 11.

From Fig. 11, the specimens could be classified into negative and positive by setting the first threshold at 1.0E + 07 copies. That is, when an expression amount of a CK19mRNA measured from a lymph node is less than the first threshold, it can be predicted that a cancer cell has not metastasized to this lymph node and, when the expression amount is the first threshold or more, it can be predicted that a cancer cell has metastasized to this lymph node.

In the present Example, the first threshold can be set to be not less than 3.0E + 06 copies which is a highest value of a background.

In addition, when a major axis of a metastatic focus is 2 mm in a tissue diagnosis, this is called macro metastasis and, when the diameter is less than 2 mm, this is called micro metastasis. Therefore, since a lowest value of an expression amount of a CK19mRNA when postulated to be a cube with one side of 2 mm is about 2.0E + 8.0 copies from Fig. 11, by setting this as the second threshold, whether the metastatic focus is micro metastasis or macro metastasis may be predicted.

### (Example 4)

To sixty four lymph nodes (negative specimen 42, positive specimen 22) taken from breast cancer patients, and sixty nine lymph nodes (negative specimen 33, positive specimen 36) taken from large intestine cancer patients (diameter: about 6 mm/lymph node) were added 4 mL of a pH 3.4 buffer (containing 200 mM glycine-HCl, 5% Brij 35 (polyoxyethylene (35) lauryl ether, Sigma) and 20% DMSO (Wako Pure Chemical Industries, Ltd.)), respectively, and this was homogenized with a blender. The homogenate was centrifuged at 10000 xg for 1 minute, and a supernatant was taken. This supernatant was 10-fold diluted with the buffer, and 2 µL of an assay sample (lysate) was taken.

This assay sample and a nucleic acid amplification reagent "Cytokeratin reagent" (manufactured by Sysmex) were set in a nucleic acid amplification apparatus "GD-100" (manufactured by Sysmex), and a CK19mRNA (copy/µL • lysate) in the assay sample was quantitated.

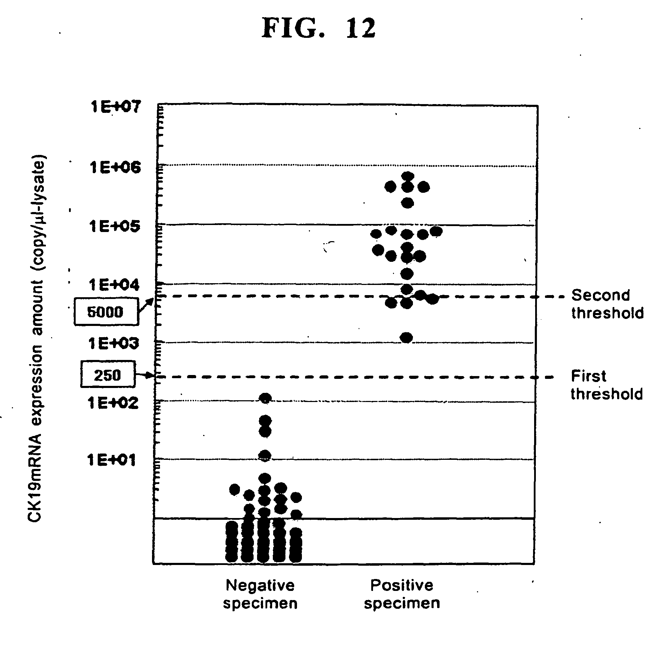

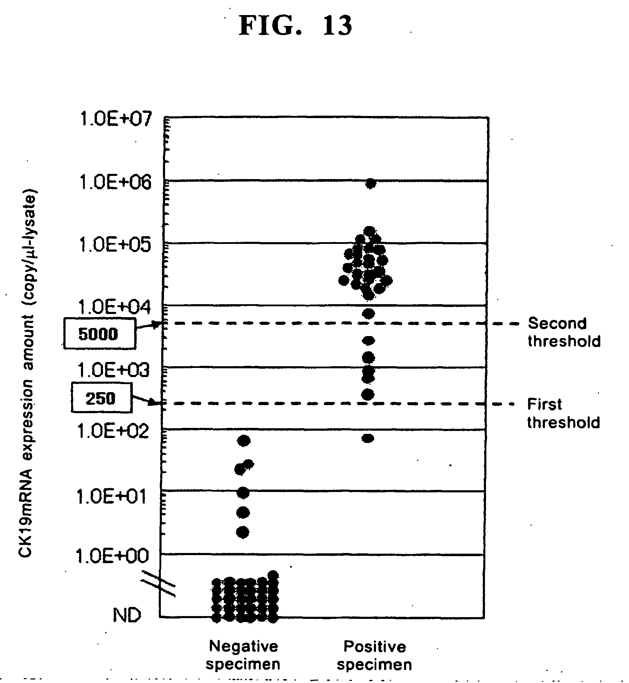

The CK19mRNA quantitation result when a lymph node taken from a breast cancer patient was used is shown in Fig. 12, and the CK19mRNA quantitation result when a lymph node taken from a large intestine cancer patient was used is shown in Fig. 13.

Since the assay sample used in the present Example was 40000-fold diluted as compared with the assay sample used in Example 3, the first threshold was set at 250 copies/µL • lysate, and the second threshold was set at 5000 copies/µL • lysate in Figs. 12 and 13.

From Fig. 12, by comparing an expression amount of a CK19mRNA (copy/µL • lysate) and the first threshold, all negative specimens could be determined to be negative, and all positive specimens could be determined to be positive. That is, it was confirmed that whether metastasis of a breast cancer to a lymph node is negative or positive could be determined at a probability of 100%. In the present Example, by setting the first threshold between 200 to 1000 copies/ µL • lysate, the similar result to that described above can be obtained.

In addition, a specimen in which an expression amount of a CK19mRNA (copy/µL • lysate) was not less than the second threshold in Fig. 12 is predicted to contain macro metastasis of a breast cancer, and a specimen in which the expression amount was not less than the first threshold and less than the second threshold is predicted to contain micro metastasis of a breast cancer.

From Fig. 13, by comparing an expression amount of a CK19mRNA (copy/µL • lysate) and the first threshold (250 copies/µL • lysate), all negative specimens could be determined to be negative, and 35 of 36 positive specimens could be determined to be positive. That is, it was confirmed that whether metastasis of a large intestine cancer to a lymph node is negative or positive could be determined at a high probability of about 99%. In the present Example, by setting the first threshold between 100 to 300 copies/µL • lysate, the similar result to that described above can be obtained.

In addition, a specimen in which an expression amount of a CK19mRNA (copy/µL • lysate) was not less than the second threshold in Fig. 13 is predicted to contain macro metastasis of a large intestine, and a specimen in which the expression amount was not less than the first threshold and less than the second threshold is predicted to contain micro metastasis of a large intestine.

From the result of the present Example, it was found that a metastatic focus in a lymph node could be measured by using the same threshold (first threshold and second threshold) in a breast cancer and a large intestine cancer.

The foregoing detailed description and examples have been provided by way of explanation and illustration, and are not intended to limit the scope of the appended claims.

## Claims

1. A method for determining a size of a metastatic focus, comprising steps of:
quantitating cytokeratin 19 mRNA in an assay sample prepared from a lymph node, and
obtaining a size of a metastatic focus in the lymph node based on the quantitation result of the mRNA.

2. The method according to claim 1, wherein the quantitation result of the mRNA is an absolute amount of the mRNA in the assay sample.

3. The method according to claim 1, further comprising a step of comparing the quantitation result of the mRNA with a threshold, and
the step of obtaining a size of a metastatic focus is performed when the quantitation result of the mRNA is the threshold or more.

4. The method according to claim 3, wherein the threshold is set at 50 to 3000 copies/µL • lysate.

5. A method for determining cancer metastasis, comprising steps of:
quantitating cytokeratin 19 mRNA in an assay sample prepared from a lymph node,
comparing the quantitation result of the mRNA with a threshold,
determining whether cancer metastasis of the lymph node is negative or positive, based on the comparison result, and
outputting information indicating that cancer metastasis of the lymph node is either of negative or positive, based on the determination result,
wherein the outputting step is performed so as to further output a size of a metastatic focus, when the determination result is positive.

6. The method according to claim 5, wherein the comparison step is performed so as to compare the quantitation result of the mRNA with the threshold and a second threshold which is higher than the threshold, and
the determination step is performed so as to determine whether cancer metastasis of the lymph node is negative, weakly positive, or strongly positive, based on the result of comparison with the threshold and the second threshold.

7. The method according to claim 6, further comprising a step of outputting information indicating whether cancer metastasis of the lymph node is negative, weakly positive, or strongly positive, based on the determination result.

8. The method according to claim 7, wherein the outputting step is performed so as to further output information indicating that a metastatic focus is micro metastasis, when the determination result is weakly positive.

9. The method according to claim 7, wherein the outputting step is performed so as to further output information indicating that a metastatic focus is macro metastasis, when the determination result is strongly positive.

10. The method according to claim 9, wherein the second threshold is set at 2000 to 20000 copies/µL • lysate.

11. An apparatus for determining the size of a metastasis focus, comprising:
a quantitation part for quantitating cytokeratin 19 mRNA in an assay sample prepared from a lymph node,
a comparison part for comparing the quantitation result of the mRNA with a threshold and a second threshold which is higher than the threshold,
a determination part for determining whether cancer metastasis of the lymph node is negative, weakly positive, or strongly positive, based on the result of comparison with the threshold and the second threshold, thereby determining the size of a metastasis focus, and
a display part for outputting information indicating whether cancer metastasis of the lymph node is negative or positive, based on the determination result,
wherein the display part further is for outputting the size of the metastatic focus, when the determination result is positive, and wherein the apparatus is suitably adapted for carrying out a method according to any one of claims 1 to 10.

12. The apparatus according to claim 11, further comprising a display part for outputting information indicating whether cancer metastasis of the lymph node is negative, weakly positive, or strongly positive, based on the determination result.

13. The apparatus according to claim 12, wherein the display part further is for outputting information indicating that a metastatic focus is micro metastasis, when the determination result is weakly positive.

14. The apparatus according to claim 12, wherein the display part further is for outputting information indicating that a metastatic focus is macro metastasis, when the determination result is strongly positive.

## Patentansprüche

1. Verfahren zur Bestimmung einer Größe eines metastatischen Fokus, umfassend die Schritte:
Quantifizieren der Cytokeratin 19-mRNA in einer Assay-Probe, die aus einem Lymphknoten präpariert wurde, und
Erhalten einer Größe eines metastatischen Fokus im Lymphknoten, basierend auf dem Quantifizierungsergebnis der mRNA.

2. Verfahren gemäß Anspruch 1, wobei das Quantifizierungsergebnis der mRNA eine absolute Menge der mRNA in der Assay-Probe ist.

3. Verfahren gemäß Anspruch 1, ferner umfassend einen Schritt des Vergleichens des Quantifizierungsergebnisses der mRNA mit einem Schwellenwert, und
der Schritt des Erhaltens einer Größe eines metastatischen Fokus wird durchgeführt, wenn das Quantifizierungsergebnis der mRNA der Schwellenwert oder mehr ist.

4. Verfahren gemäß Anspruch 3, wobei der Schwellenwert auf 50 bis 3.000 Kopien/µl · Lysat gesetzt ist.

5. Verfahren zur Bestimmung von Krebs-Metastasierung, umfassend die Schritte:
Quantifizieren der Cytokeratin 19-mRNA in einer Assay-Probe, die aus einem Lymphknoten präpariert wurde,
Vergleichen des Quantifizierungsergebnisses der mRNA mit einem Schwellenwert,
Bestimmen, ob Krebs-Metastasierung des Lymphknotens negativ oder positiv ist, basierend auf dem Ergebnis des Vergleichs, und
das Ausgeben von Information(en), die anzeigt/anzeigen, dass Krebs-Metastasierung des Lymphknotens entweder negativ oder positiv ist, basierend auf dem Ergebnis der Bestimmung,
wobei der Schritt des Ausgebens so durchgeführt wird, dass ferner eine Größe eines metastatischen Fokus ausgegeben wird, wenn das Ergebnis der Bestimmung positiv ist.

6. Verfahren gemäß Anspruch 5, wobei der Vergleichsschritt so durchgeführt wird, dass das Quantifizierungsergebnis der mRNA mit dem Schwellenwert und einem zweiten Schwellenwert, der höher als der Schwellenwert ist, verglichen wird, und
der Bestimmungsschritt so durchgeführt wird, dass bestimmt wird, ob Krebs-Metastasierung des Lymphknotens negativ, schwach positiv oder stark positiv ist, basierend auf dem Ergebnis des Vergleichs mit dem Schwellenwert und dem zweiten Schwellenwert.

7. Verfahren gemäß Anspruch 6, ferner umfassend einen Schritt der Ausgabe von Information(en), die anzeigt/anzeigen, ob Krebs-Metastasierung des Lymphknotens negativ, schwach positiv oder stark positiv ist, basierend auf dem Ergebnis der Bestimmung.

8. Verfahren gemäß Anspruch 7, wobei der Schritt der Ausgabe so durchgeführt wird, dass ferner Information(en) ausgegeben wird/werden, die anzeigt/anzeigen, dass ein metastatischer Fokus eine Mikrometastase ist, wenn das Ergebnis der Bestimmung schwach positiv ist.

9. Verfahren gemäß Anspruch 7, wobei der Schritt der Ausgabe so durchgeführt wird, dass ferner Information(en) ausgegeben wird/werden, die anzeigt/anzeigen, dass ein metastatischer Fokus eine Makrometastase ist, wenn das Ergebnis der Bestimmung stark positiv ist.

10. Verfahren gemäß Anspruch 9, wobei der zweite Schwellenwert auf 2.000 bis 20.000 Kopien/µl · Lysat gesetzt ist.

11. Vorrichtung zur Bestimmung der Größe eines Metastase-Fokus, umfassend:
einen Quantifizierungsteil zur Quantifizierung der Cytokeratin 19-mRNA in einer Assay-Probe, die aus einem Lymphknoten präpariert wurde,
ein Vergleichsteil zum Vergleichen des Quantifizierungsergebnisses der mRNA mit einem Schwellenwert und einem zweiten Schwellenwert, der höher ist als der Schwellenwert,
einen Bestimmungsteil, um zu bestimmen, ob Krebs-Metastasierung des Lymphknotens negativ, schwach positiv oder stark positiv ist, basierend auf dem Ergebnis des Vergleichs mit dem Schwellenwert und dem zweiten Schwellenwert, wodurch die Größe eines Metastase-Fokus bestimmt wird, und
einen Display-Teil zum Ausgeben von Information(en), die anzeigt/anzeigen, ob Krebs-Metastasierung des Lymphknotens negativ oder positiv ist, basierend auf dem Ergebnis der Bestimmung,
wobei der Display-Teil ferner zum Ausgeben der Größe des metastatischen Fokus da ist, wenn das Ergebnis der Bestimmung positiv ist, und wobei die Vorrichtung in geeigneter Weise angepasst ist, um ein Verfahren gemäß einem der Ansprüche 1 bis 10 durchzuführen.

12. Vorrichtung gemäß Anspruch 11, ferner umfassend einen Display-Teil zum Ausgeben von Information(en), die anzeigt/anzeigen, ob Krebs-Metastasierung des Lymphknotens negativ, schwach positiv oder stark positiv ist, basierend auf dem Ergebnis der Bestimmung.

13. Vorrichtung gemäß Anspruch 12, wobei der Display-Teil ferner da ist, um Information(en) auszugeben, die anzeigt/anzeigen, dass ein metastatischer Fokus eine Mikrometastase ist, wenn das Ergebnis der Bestimmung schwach positiv ist.

14. Vorrichtung gemäß Anspruch 12, wobei der Display-Teil ferner da ist, um Information(en) auszugeben, die anzeigt/anzeigen, dass ein metastatischer Fokus eine Makrometastase ist, wenn das Ergebnis der Bestimmung stark positiv ist.

## Revendications

1. Procédé pour déterminer la taille d'un foyer métastatique, comprenant les étapes consistant à :
quantifier l'ARNm de la cytokératine 19 dans un échantillon de test préparé à partir d'un ganglion lymphatique, et
obtenir la taille d'un foyer métastatique dans le ganglion lymphatique en se basant sur le résultat de la quantification de l'ARNm.

2. Procédé selon la revendication 1, dans lequel le résultat de la quantification de l'ARNm est une quantité absolue de l'ARNm dans l'échantillon de test.

3. Procédé selon la revendication 1, comprenant en outre une étape consistant à comparer le résultat de la quantification de l'ARNm avec une valeur seuil, et
l'étape consistant à obtenir la taille d'un foyer métastatique est réalisée lorsque le résultat de la quantification de l'ARNm est la valeur seuil ou davantage.

4. Procédé selon la revendication 3, dans lequel la valeur seuil est définie à 50 à 3000 copies/µl de lysat.

5. Procédé pour déterminer une métastase cancéreuse, comprenant les étapes consistant à :
quantifier l'ARNm de la cytokératine 19 dans un échantillon de test préparé à partir d'un ganglion lymphatique,
comparer le résultat de la quantification de l'ARNm avec une valeur seuil,
déterminer si la métastase cancéreuse du ganglion lymphatique est négative ou positive, en se basant sur le résultat de la comparaison, et
émettre des informations indiquant que la métastase cancéreuse du ganglion lymphatique est soit négative soit positive, en se basant sur le résultat de la détermination,
où l'étape d'émission d'informations est réalisée de sorte à en outre émettre la taille d'un foyer métastatique, lorsque le résultat de la détermination est positif.

6. Procédé selon la revendication 5, dans lequel l'étape de comparaison est réalisée de sorte à comparer le résultat de la quantification de l'ARNm avec la valeur seuil et une deuxième valeur seuil qui est supérieure à cette valeur seuil, et
l'étape de détermination est réalisée de sorte à déterminer si la métastase cancéreuse du ganglion lymphatique est négative, faiblement positive, ou fortement positive, en se basant sur le résultat de la comparaison avec la valeur seuil et la deuxième valeur seuil.

7. Procédé selon la revendication 6, comprenant en outre une étape consistant à émettre des informations indiquant si la métastase cancéreuse du ganglion lymphatique est négative, faiblement positive, ou fortement positive, en se basant sur le résultat de la détermination.

8. Procédé selon la revendication 7, dans lequel l'étape d'émission d'informations est réalisée de sorte à en outre émettre des informations indiquant qu'un foyer métastatique est une micro-métastase, lorsque le résultat de la détermination est faiblement positif.

9. Procédé selon la revendication 7, dans lequel l'étape d'émission d'informations est réalisée de sorte à en outre émettre des informations indiquant qu'un foyer métastatique est une macro-métastase, lorsque le résultat de la détermination est fortement positif.

10. Procédé selon la revendication 9, dans lequel la deuxième valeur seuil est définie à 2000 à 20 000 copies/µl de lysat.

11. Appareil pour déterminer la taille d'un foyer métastatique, comprenant :
une partie de quantification pour quantifier l'ARNm de la cytokératine 19 dans un échantillon de test préparé à partir d'un ganglion lymphatique,
une partie de comparaison pour comparer le résultat de la quantification de l'ARNm avec une valeur seuil et une deuxième valeur seuil qui est supérieure à cette valeur seuil,
une partie de détermination pour déterminer si la métastase cancéreuse du ganglion lymphatique est négative, faiblement positive, ou fortement positive, en se basant sur le résultat de la comparaison avec la valeur seuil et la deuxième valeur seuil, ce qui permet ainsi de déterminer la taille d'un foyer métastatique, et
une partie d'affichage pour émettre des informations indiquant si la métastase cancéreuse du ganglion lymphatique est négative ou positive, en se basant sur le résultat de la détermination,
où la partie d'affichage est en outre destinée à émettre la taille du foyer métastatique, lorsque le résultat de la détermination est positif, et où l'appareil est convenablement adapté pour mettre en oeuvre un procédé selon l'une quelconque des revendications 1 à 10.

12. Appareil selon la revendication 11, comprenant en outre une partie d'affichage pour émettre des informations indiquant si la métastase cancéreuse du ganglion lymphatique est négative, faiblement positive, ou fortement positive, en se basant sur le résultat de la détermination.

13. Appareil selon la revendication 12, dans lequel la partie d'affichage est en outre destinée à émettre des informations indiquant qu'un foyer métastatique est une micro-métastase, lorsque le résultat de la détermination est faiblement positif.

14. Appareil selon la revendication 12, dans lequel la partie d'affichage est en outre destinée à émettre des informations indiquant qu'un foyer métastatique est une macro-métastase, lorsque le résultat de la détermination est fortement positif.


### SEQUENCE LISTING

<110> SYSMEX Corporation
<120> A method and apparatus for measuring carcinomatous lesion
<130> 06-060
<160> 3
<170> PatentIn version 3.1
<210> 1
   <211> 20
   <212> DNA
   <213> Artificial
<220>
   <223> Designed primer for CK19 mRNA
<400> 1
   cagatcgaag gcctgaagga 20
<210> 2
   <211> 19
   <212> DNA
   <213> Artificial
<220>
   <223> Designed primer for CK19 mRNA
<400> 2
   cttggcccct cagcgtact 19
<210> 3
   <211> 30
   <212> DNA
   <213> Artificial
<220>
   <223> Designed probe for CK19 mRNA
<400> 3
   gcctacctga agaagaacca tgaggaggaa 30